(19) Europäisches Patentamt / European Patent Office / Office européen des brevets

(11) **EP 1 782 775 A1**

(12) **EUROPEAN PATENT APPLICATION**
published in accordance with Art. 158(3) EPC

(43) Date of publication:
09.05.2007 Bulletin 2007/19

(51) Int Cl.:
*A61F 7/08* (2006.01)    *C09K 5/16* (2006.01)

(21) Application number: 05765768.6

(22) Date of filing: 14.07.2005

(86) International application number:
PCT/JP2005/013004

(87) International publication number:
WO 2006/006651 (19.01.2006 Gazette 2006/03)

(84) Designated Contracting States:
DE FR GB

(30) Priority: 14.07.2004 JP 2004207832

(71) Applicant: Mycoal Products Corporation
Tochigi-chi, Tochigi 328-0067 (JP)

(72) Inventor: DODO, Toshihiro,
MYCOAL PRODUCTS CORPORATION
Tochigi-shi, Tochigi 328-0067 (JP)

(74) Representative: Thun, Clemens et al
Mitscherlich & Partner
Sonnenstrasse 33
80331 München (DE)

(54) **HEAT GENERATING BODY AND METHOD OF PRODUCING THE SAME**

(57)    There are provided a heat generating body having an exothermic part made of sectional exothermic parts in a stripe form, which is thin and flexible; even when as a reaction of an air-permeable exothermic heat generating body proceeds, a heat generating composition becomes massive so that flexibility is lowered, or a part of an accommodating bag rides up by shrinkage and curling as caused by heat generation, well keeps the bonding and holding state so that it does not fall off easily; is supple like cloths; is excellent in flexing properties; easily and surely fits to flexible places such as elbows and knees; is able to take warmth; is applicable with good follow-up deformation properties to various places of a human body such as curved parts including shoulders, arms, a neck and feet; and hardly causes an uncomfortable feeling and a process for producing the same.

A heat generating body having an exothermic part in a stripe form of an integral structure made of a sectional exothermic part as formed by providing plural heat generating composition molded bodies in a stripe form at intervals on a planar substrate, covering a covering material as folded in a wavy shape thereon such that crests of the covering material wrap the heat generating composition molded bodies and sealing the peripheries of the heat generating composition molded bodies and a sectioned part as the seal part, which is **characterized in that** the heat generating composition molded body is constituted of a moldable heat generating composition; the moldable heat generating composition contains, as essential components, an iron powder, a carbon component, a reaction accelerator and water but not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air; a ratio of the height of a convex of the covering material in a wavy shape to the height of the heat generating composition molded body and a ratio of the width of a convex of the covering material in a wavy shape to the width of the heat generating composition molded body are from 0.7 to 1.0, respectively; a part of the sectional exothermic part is air-permeable; and the surroundings of the heat generating body are sealed.

EP 1 782 775 A1

# FIG. 2

**Description**

[Technical Field]

The present invention relates to a heat generating body having an exothermic part in a stripe form, which is excellent in flexing properties, easily and surely fits to flexible places such as elbows and knees, is able to take warmth, is applicable with good follow-up deformation properties to various places of a human body such as curved parts including shoulders, arms, a neck and feet and hardly causes an uncomfortable feeling, the heat generating body being produced by using a covering material as previously folded in a wavy shape, covering the covering material so as to wrap a heat generating composition molded body laminated in a stripe form on a substrate and sealing at least the periphery of the heat generating composition molded body, and to a process for producing the same.

[Background Art]

Heat generating compositions utilizing an oxidation reaction of a metal such as iron have been provided as a powder or granule, or a viscous material or creamy material. Heat generating bodies utilizing such a heat generating composition are very excellent in view of costs, safety, exothermic temperature, and so on and are already put into practical use as, for example, a chemical body warmer as filled in an air-permeable bag.

In order to obtain a more comfortable feeling for use, there have been proposed various heat generating compositions which design to have shape holding properties and to hold exothermic characteristics while using a thickener, a binding agent, etc. in quest of prevention of deviation of a heat generating composition and fitness to various kinds of shapes.

For example, Patent Document 1 proposes a process for producing a heat generating composition as granulated so as to have an average particle size of 0.5 mm or more and a process for producing a heat generating composition having an improved granular strength by blending from 10 to 20 parts by weight of an adhesive binder component in addition water and granulating.

Also, Patent Document 2 proposes a throwaway body warmer composed of a heat generating composition having shape holding characteristics by adding a powdered thickener such as corn starch and potato starch.

Also, Patent Document 3 proposes a solid heat generating composition as prepared by mixing a binding agent such as CMC in a powdered or granular heat generating composition and compression molding the mixture.

Also, Patent Document 4 proposes a heat generating body as prepared by using a crosslinking agent, etc. and a water absorptive polymer and integrating them under pressure. Also, Patent Document 5 proposes a heat generating composition in the range of from an ink form to a creamy form using a thickener so as to have viscosity, a heat generating body and a process for producing the same.

Also, Patent Document 6 proposes a heat generating composition molded body using a binding agent, the surface of which is covered by an air-permeable film material such as CMC, thereby designing to hold the shape.

Also, Patent Document 7 and Patent Document 8 propose a heat generating composition as processed into a viscous material or a creamy material, in which the shape is changed from a conventional rectangle to a foot shape or an elliptical shape so as to adapt to the outline of a body to be warmed.

Also, Patent Document 9, Patent Document 10, Patent Document 11, Patent Document 12 and Patent Document 13 each proposes a heat generating composition using a flocculant aid and a dry binding agent and a heat generating body in which a heat generating composition exothermic part is sectioned into plural divisions by using a substrate having an accommodating pocket for the purpose of increasing fitness to the body or the like.

However, following spreading in utilization of throwaway body warmers which are aimed to be applied to various places of a human body such as shoulders, arms, a neck and feet, even if a heat generating composition is hardened by a thickener, etc., there were encountered problems that in a single packed state, for example, bonding retention is difficult so that the dropping easily occurs and that a strong uncomfortable feeling is caused in wearing. Such problems are promoted due to a lowering of flexibility as caused by blocking following progression of a reaction of heat generating body. There was also encountered a problem that a stretched film which forms an accommodating bag is shrunk and curled due to heat generation so that an end part of a single packaging bag rides up, whereby a body warmer as bonded and held easily peels away and drops due to catch therein.

Furthermore, even if the exothermic part is divided into plural compartments, a heat generating composition as hardened by a flocculant aid, etc. involved a problem that an exothermic performance is deteriorated.

Furthermore, so far, a heat generating body was produced by a filling system or produced by filling a heat generating composition containing a flocculant and a binding agent in a packaging material having accommodating divisions as molding in vacuo an agglomerate or compressed body. Moreover, a heat generating body was produced by previously preparing a filling pocket in a substrate, filling a heat generating composition in the pocket and covering a packaging material thereon, followed by sealing.

Furthermore, in the case of producing a heat generating body having sectioned exothermic parts by using a powdered heat generating composition or a granular heat generating composition as a heat generating composition, according to a method using a filling system, since the powdered heat generating composition or granular heat generating composition is accommodated in an accommodating body in a partially sealed bag form and the whole is then sealed, there was a limit in size of a sectional region in view of the production. That is, according to a method for filling a powdered heat generating composition or a granular heat generating composition while partially sealing, it was mechanically substantially impossible to produce a heat generating body having a plural number of small-sized sectional regions, and additionally, there was caused a problem due to shortage in sealing as caused by incorporation of the heat generating composition into a seal part or the like. In particular, it was substantially impossible to continuously produce one having a partial shape having a size of not more than 20 mm or one having a small shape of not more than 20 mm. Furthermore, according to a method using a rotary magnet system, not only a complicated operation is necessary, but also its structure is complicated. Accordingly, there were encountered problems that the operation at the time of forming an exothermic layer is troublesome and that a device to be used is complicated and expensive, is liable to cause a fault, takes a long time to do the maintenance and is inconvenient for handling. Therefore, there was a limit in making the size of the exothermic part small.

Furthermore, according to a method using a pocket system, a heat generating composition containing a flocculant and a binding agent is used and a dry powdered mixture of an exothermic component containing a flocculant and a binding agent is filled in a concave pocket as previously prepared in a packaging material directly or after compressing it to form a granule, a pellet, a tablet or a scrub, followed by compression to prepare an exothermic part. Now, in comparison with a heat generating body in which a flocculant and a binding agent are not incorporated, in a heat generating body having plural sectional exothermic parts, the exothermic time is markedly short, especially, in a sectional exothermic part having a narrow region whose shortest length is not more than 15 mm or a sectional exothermic part of a small size, the exothermic duration is markedly short, resulting in a problem in view of practical use. If it is intended to prolong the exothermic duration, there was a problem that it becomes necessary to increase the dimensions of one sectioned exothermic part so that the heat generating body becomes one having a sectional exothermic part of a large size. Furthermore, even by using a powdered or granular heat generating composition, there was a problem that a concave pocket must be previously provided in a packaging material, thereby bringing a complicated operation.

[Patent Document 1] JP-A-4-293989
[Patent Document 2] JP-A-6-343658
[Patent Document 3] JP-A-59-189183
[Patent Document 4] WO 00/13626
[Patent Document 5] JP-A-9-75388
[Patent Document 6] JP-A-60-101448
[Patent Document 7] JP-A-9-276317
[Patent Document 8] JP-A-11-299817
[Patent Document 9] JP-UM-A-6-26829
[Patent Document 10] JP-A-2000-288008
[Patent Document 11] JP-T-11-508314
[Patent Document 12] JP-T-11-508786
[Patent Document 13] JP-T-2002-514104

[Disclosure of the Invention]

[Problems that the Invention is to Solve]

The invention is to provide a heat generating body having an exothermic part made of sectional exothermic parts in a stripe form, which is thin and flexible; even when as a reaction of an air-permeable exothermic heat generating body proceeds, a heat generating composition becomes massive so that flexibility is lowered, or a part of an accommodating bag rides up by shrinkage and curling as caused by heat generation, well keeps the bonding and holding state so that it does not fall off easily; is supple like cloths; is excellent in flexing properties; easily and surely fits to flexible places such as elbows and knees; is able to take warmth; is applicable with good follow-up deformation properties to various places of a human body such as curved parts including shoulders, arms, a neck and feet; and hardly causes an uncomfortable feeling and a process for producing the same.

[Means for Solving the Problems]

As set forth in claim 1, a heat generating body of the invention is a heat generating body having an exothermic part in a stripe form of an integral structure made of a sectional exothermic part as formed by providing plural heat generating composition molded bodies in a stripe form at intervals on a planar substrate, covering a covering material as folded in a wavy shape thereon such that crests of the covering material wrap the heat generating composition molded bodies and sealing the peripheries of the heat generating composition molded bodies and a sectioned part as the seal part, which is
characterized in that:

1) the heat generating composition molded body is constituted of a moldable heat generating composition,
2) the moldable heat generating composition contains, as essential components, an iron powder, a carbon component, a reaction accelerator and water but not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air,
3) a ratio of the height of a convex of the covering material in a wavy shape to the height of the heat generating composition molded body and a ratio of the width of a convex of the covering material in a wavy shape to the width of the heat generating composition molded body are from 0.7 to 1.0, respectively,
4) a part of the sectional exothermic part is air-permeable, and
5) the surroundings of the heat generating body are sealed.
Also, a heat generating body as set forth in claim 2 is

characterized in that in the heat generating body as set forth in claim 1, a volume of the heat generating composition molded body is from 0.1 to 30 $cm^3$; a ratio of the capacity of the sectional exothermic part to the volume of the heat generating composition molded body is from 0.6 to 1.0; a maximum height of the sectional exothermic part is from 0.1 to 10 mm; a width of the sectioned part is from 0.3 to 50 mm; and the seal part is formed by heat sealing.
Also, a heat generating body as set forth in claim 3 is characterized in that in the heat generating body as set forth in claim 1, the moldable heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.
Also, a heat generating body as set forth in claim 4 is characterized in that in the heat generating body as set forth in claim 1, in the heat generating body, the heat generating composition molded body is at least compressed.
Also, a heat generating body as set forth in claim 5 is
characterized in that in the heat generating body as set forth in claim 1, a fixing measure is provided in at least a part of the exposed surface of the heat generating body.
As set forth in claim 6, a process for producing a heat generating body of the invention is characterized in that:

1) at least one of a substrate and a covering material is air-permeable,
2) a composition which contains, as essential components, an iron powder, a carbon component, a reaction accelerator and water but does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air is used as a moldable heat generating composition,
3) plural heat generating composition molded bodies as formed by molding the moldable heat generating composition are provided in a stripe form at intervals on the substrate,
4) the covering material is formed in a wavy shape by a fold-providing machine and covered such that crests of the covering material come into contact with the heat generating molded body,
5) the substrate covered with covering material is conveyed while a part to be sealded is pressed by a pressing tool, and
6) at least the periphery of the heat generating composition molded body is sealed in the MD direction while pressing both end parts of the covering material.
Also, a process for producing a heat generating body as set forth in claim 6 is characterized in that in the process for producing a heat generating body having a sectional exothermic part in a stripe form as set forth in claim 1, folding is initiated from the central part of the covering material and carried out in succession towards the end parts. Also, a process for producing a heat generating body as set forth in claim 6 is characterized in that in the process for producing a heat generating body as set forth in claim 7, a volume of the heat generating composition molded body is from 0.1 to 30 cm$^3$; a ratio of the capacity of the sectional exothermic part to the volume of the heat generating composition molded body is from 0.6 to 1.0; a maximum height of the sectional exothermic part is from 0.1 to 10 mm; and a width of the sectioned part is from 0.3 to 50 mm.

Also, a process for producing a heat generating body as set forth in claim 7 is characterized in that in the process for producing a heat generating body as set forth in claim 1, the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, in the foregoing heat generating body, it is preferable that the moldable heat generating composition contains components resulting from a contact treatment of a mixture at least containing, as essential components, an iron powder, a carbon component, a reaction accelerator and water with an oxidizing gas.

Also, in the foregoing heat generating body, it is preferable that the iron powder is covered by an iron oxide film on at least a part of the surface thereof; that the oxide film has a thickness of 3 nm or more; and that from 20 to 100 % by weight of an active iron powder having a region of an oxygen-free iron component is contained in at least one region selected from a central region of the iron powder and a region beneath of the iron oxide film.

Also, in the foregoing heat generating body, it is preferable that the iron powder is covered by a wustite film on at least a part of the surface thereof; and that from 20 to 100 % by weight of an active iron powder having an amount of wustite of from 2 to 50 % by weight in terms of an X-ray peak intensity ratio to iron is contained.

Also, in the foregoing heat generating body, it is preferable that a perforation is provided in the sectioned part.

Also, in the foregoing heat generating body, it is preferable that the fixing measure is an adhesive layer; and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

Also, in the foregoing process for producing a heat generating body, it is preferable that the sealing is heat sealing.

Also, in the foregoing process for producing a heat generating body, it is preferable that the sealing is carried out by heat sealing after contact bond sealing.

[Advantages of the Invention]

As is clear from the foregoing description, there are brought the following advantages.

1. Since a covering material as previously folded in a wavy shape is used, the covering material is covered so as to wrap a heat generating composition molded body as laminated in a stripe form on a substrate and at least the periphery of the heat generating composition molded body is sealed, the heat generating composition molded body can be surely covered and sealed and high-speed production can be achieved without causing seal failure.

2. Since folds in a wavy shape are previously provided in the covering material, the covering material is covered on two or more plural heat generating composition molded bodies as laminated in a stripe form at intervals on a planar substrate such that crests of the covering material as previously folded in a wavy shape wrap the heat generating composition molded bodies and at least the periphery of the heat generating composition molded body is sealed, heat sealing can be surely achieved so that it has become possible to realize high speed of heat sealing and to make the heat seal width thin.

3. The width of the heat seal part which sections an exothermic part can be made thin, a distance between sectional exothermic parts becomes narrow so that a heat insulation effect can be mutually kept, and the exothermic part can be sectioned without causing a lowering of exothermic characteristics such as a lowering in exothermic time due to sectioning of the exothermic part, whereby it has become possible to obtain a flexible heat generating body having excellent exothermic characteristics.

In the light of the above, the invention is concerned with a heat generating body having an exothermic part in a stripe form using a heat generating composition molded body resulting from molding a moldable heat generating composition containing surplus water as a connecting substance, in which the heat generating composition does not use a flocculant aid, a dry binding agent, a flocculant, and so on but contains, as a connecting substance, a suitable amount of surplus water as expressed in terms of a water mobility value. Also, a covering material as previously folded in a wavy shape is used and covered such that crests thereof wrap the heat generating composition molded body, and a valley part is sealed. Thus, the heat generating composition molded body can be surely accommodated without causing cutting in seal even in an irregular state. Also, by constituting the exothermic part from a sectional exothermic part in a stripe form, fitness of the heat generating body to a body to be worn has been remarkably enhanced.

[Best Modes for Carrying Out the Invention]

The heat generating body of the invention is a heat generating body having an exothermic part in a stripe form of an integral structure made of a sectional exothermic part as formed by providing plural heat generating composition molded bodies in a stripe form at intervals on a planar substrate, covering a covering material as folded in a wavy shape thereon such that crests of the covering material wrap the heat generating composition molded bodies and sealing the peripheries of the heat generating composition molded bodies and a sectioned part as the seal part, which is characterized in that:

1) the heat generating composition molded body is constituted of a moldable heat generating composition,
2) the moldable heat generating composition contains, as essential components, an iron powder, a carbon component, a reaction accelerator and water but not containing a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air,
3) a ratio of the height of a convex of the covering material in a wavy shape to the height of the heat generating composition molded body and a ratio of the width of a convex of the covering material in a wavy shape to the width of the heat generating composition molded body are from 0.7 to 1.0, respectively,
4) a part of the sectional exothermic part is air-permeable, and
5) the surroundings of the heat generating body are sealed.

In the heat generating body of the invention, since heat sealing is carried out at high speed, the heat seal width is made thin and heat sealing is carried out surely, there has been developed a method in which folds in a wavy shape are previously provided in the covering material, the covering material is covered on two or more plural heat generating composition molded bodies as laminated in a stripe form at intervals on a planar substrate such that crests of the covering material as previously folded in a wavy shape wrap the heat generating composition molded bodies and at least the periphery of the heat generating composition molded body is sealed.

In this way, the width of the heat seal part which sections an exothermic part can be made thin, a distance between sectional exothermic parts becomes narrow so that a heat insulation effect can be mutually kept, and the exothermic part can be sectioned without causing a lowering of exothermic characteristics such as a lowering in exothermic time due to sectioning of the exothermic part, whereby it has become possible to obtain a flexible heat generating body having excellent exothermic characteristics.

In the case of a molding system of the invention, with respect to the molding order, the size of the heat generating composition molded body is determined, and the size of the sectional exothermic part is then determined.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm. A capacity of the sectional exothermic part or a volume of the heat generating composition molded body is usually from 0.015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0.6 to 1, preferably from 0.7 to 1, more preferably from 0.8 to 1, and further preferably from 0.9 to 1.0. Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, the "volume of the heat generating composition molded body of the invention" as referred to herein means a volume of the heat generating composition molded body or compressed heat generating composition molded body. Furthermore, the "capacity of the sectional exothermic part" as referred to herein means an internal capacity of the sectional exothermic part having a heat generating composition molded body accommodated therein.

Furthermore, the sectioning can be formed in arbitrary directions such as a length or width direction, length and width directions, and an oblique direction.

With respect to the shape of each of the heat generating composition molded body and the exothermic part, any shape may be employed so far as it is in a stripe form. Examples of a planar shape include a rectangular shape; and examples of a steric shape include a rectangular parallelepiped shape, an oblong parallelepiped shape, a cylindrical shape, a semi-pillar shape, a semi-elliptical shape, a semi-cylindrical shape, a pillar shape, and an elliptical shape. Furthermore, such a shape may be formed in a curved surface shape by rounding the corners thereof. Furthermore, a concave may be present in the central part or the like of the heat generating composition molded body. In the invention, it is to be noted that a heat generating composition compressed body which is a compressed heat generating composition molded body is included in the heat generating composition molded body, too.

In the sectional exothermic part, the accommodating bag, the outer bag (accommodating bag of the heat generating body), and the like, packaging materials or the like constituting the same are sealed in the sectioned part as a seal part or its periphery or surroundings. Though heat seal is usually employed, other seal method can be employed depending upon the utility. As one example, sealing is carried out in a point-like (intermittent) manner or entirely by compression seal (adhesive seal), warm compression seal (adhesive seal), bonding seal, heat bonding seal, heat melt seal (heat seal), etc. by means of pressurizing, warming, heating or a combination thereof via an adhesive layer and/or a bonding agent layer and/or a heat seal layer. Selection of any one or a combination of these methods may be made depending upon the desire. In this way, it is possible to seal and form a sectional exothermic part, an inner bag (accommodating bag), an outer bag, etc. Sewing processing can also be employed as one of seal means.

A heat generating body in which a number of sectional exothermic parts are continuously provided in a stripe form and a perforation from which cutting by hand is possible is provided in the sectioned part in a stripe form can be cut into an appropriate size at the time of use on the basis of the purpose for use adaptive to a place for application of a human body, or the like and applied. In that case, the size of the heat generating body and the size and number of the sectional exothermic parts may be properly set up. There are no limitations regarding such size and number. Furthermore, the sectioned part can be formed in arbitrary directions such as a length or width direction, length and width directions, and an oblique direction.

Furthermore, at least one surface of the heat generating body having a sectional exothermic part in a stripe form may be covered by a covering material. As the packaging materials, raw materials which are used in the substrate, the covering material and the underlay material can be used.

For example, in the case of producing the heat generating body of the invention by using a film having a heat seal layer as the packaging material, when the heat generating body of the invention is produced from a heat generating composition molded body as prepared by previously folding a perforated heat sealable plastic film as an air-permeable packaging material in a wavy shape, using this plastic film as a covering material and laminating it on a substrate, a non-woven fabric is stuck onto the air-permeable side thereof by an air-permeable adhesive layer, the heat insulation at the time of use or the prevention of leakage of collapsed pieces of the heat generating composition from occurring may be achieved. Also, a heat generating body for thermal muffler may be formed by wrapping the both surfaces of the foregoing heat generating body by a packaging material such as non-woven fabrics.

At least a part of the surface of the heat generating composition molded body may be covered by an air-permeable adhesive layer made of a net-work hot melt based adhesive, etc. , or an underlay material such as a non-woven fabric may be provided between the air-permeable adhesive layer and the covering material.

Furthermore, at least one member of the heat generating composition molded body, the substrate, the covering material, the air-permeable adhesive layer, and the underlay material may be entirely or partly subjected to a pressurizing treatment or provided with irregularities. In this may, the transfer of the heat generating composition molded body between the substrate and the covering material may be prevented.

That is, a molded body prepared by appropriately compressing the heat generating composition molded body of the invention under pressure is markedly improved in moldability. For example, even when a perforated film which is difficult with respect to the pressure adjustment is used as a raw material of the air-permeable part in place of the porous film, in the case where an inner pressure of the accommodating bag becomes equal to or more than the outer pressure, shape collapse hardly occurs so that the use of a perforated film is possible. Accordingly, not only the range for selecting an air-permeable raw material is widened so that the costs can be lowered, but also a body to be warmed can be uniformly warmed at an appropriate temperature over a long period of time.

Also, in the exothermic part, by containing a magnetic substance in at least a part thereof or one sectional exothermic part, it is possible to accommodate a magnetic substance such as a magnet for the purpose of improving circulation of the blood or improving stiffness of the shoulders due to a magnetic effect.

With respect to the shape of the heat generating body, any shape may be employed. Examples of a planar shape include a circular shape, an elliptical shape, a star-like shape, a triangular shape, a square shape, a rectangular shape, a trapezoidal shape, a rhombic shape, a pentagonal or polygonal shape, a crescent shape, a broad bean-like shape, a cocoon-like shape, a gourd-like shape, a rectangular shape with rounded corners, a square shape with rounded corners, an egg-like shape, a boomerang-like shape, a comma-shaped bead-like shape, a wing-like shape, and a nose-like shape. Incidentally, the corners of the heat generating body may be trimmed in a curved line or curved surface shape by rounding.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material.

Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and *dl*-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The content of this drug is not particularly limited so far as it falls within the range where medicinal properties can be expected. However, the content of a percutaneously absorptive drug is preferably from 0.01 to 25 parts by weight, and more preferably from 0.5 to 15 parts by weight based on 100 parts by weight of the adhesive from the viewpoints of pharmacological effect, economy, and adhesive strength, and so on.

Furthermore, at least one or a part of the substrate, the covering material, the air permeability adjusting material, the adhesive layer, and the separator, each of which constitutes the heat generating body, may be provided with at least one member of characters, designs, symbols, numerals, patterns, photographs, pictures, and colors.

The substrate, the covering material, the air permeability adjusting material, and the adhesive layer, each of which constitutes the heat generating body, may be transparent, opaque, colored, or colorless. Furthermore, a layer constituting at least one layer of the layers constituting the respective materials and layers may be colored to a color different from those of other layers.

The heat generating composition is not limited so far as it is a heat generating composition which contains, as essential components, an iron powder, a carbon component, a reaction accelerator and water but does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water, with the water in the heat generating composition not functioning as a barrier layer, and is capable of causing a heat reaction upon contact with air.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth) acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly(meth)acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, $NaOH$, $KOH$, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol·ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film , and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed.

The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;

(B) an active iron powder in which the content of wustite is from 2 to 50 % by weight in terms of an X-ray peak intensity ratio to iron;

(C) an iron powder having an iron oxide film having a thickness of 3 nm or more on the surface thereof; and

(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu$m or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0.5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.

Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;

(2) a process for producing a heat generating mixture by subjecting a reaction mixture of an iron powder, a reaction accelerator, an acidic substance and water in an oxidizing gas atmosphere to a self-exothermic reaction;

(3) a process for producing a heat generating mixture by subjecting a reaction mixture of an iron powder, a reaction accelerator, a carbon component and water in an oxidizing gas atmosphere to a self-exothermic reaction;

(4) a process for producing a heat generating mixture by subjecting a reaction mixture of an iron powder, a reaction accelerator, an acidic substance, a carbon component and water in an oxidizing gas atmosphere to a self-exothermic reaction;

(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;

(6) a process for producing a heat generating mixture by carrying out the method as set forth above in any one of (1) to (5) under circumstances heated so as to have temperature of at least 10 °C higher than the circumferential temperature;

(7) a process for producing a heat generating mixture by carrying out the method as set forth above in any one of (1) to (6) by blowing an oxidizing gas;

(8) a process for producing a heat generating mixture by carrying out the method as set forth above in (7) by blowing the oxidizing gas heated so as to have a temperature of at least 10 °C higher than the circumferential temperature;

(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;

(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;

(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and

(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.

Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);

2) a heat generating composition obtained by adding other components to the heat generating composition as set forth above in 1); and

3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used. The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.
2) A magnet is provided in the vicinity of a central part of the back side of a polyvinyl chloride-made supporting plate (3 mm in thickness×600 mm in length×600 mm in width) of a footed supporting table so as to cover a cavity shape of a molding die.
3) A temperature sensor is placed on the central part of the supporting plate.
4) A polyethylene film (25 $\mu$m in thickness×250 mm in length×200 mm in width) as provided with an adhesive layer having a thickness of about 80 $\mu$m is stuck onto the supporting plate via a sticky layer such that the center of the polyethylene film is positioned at the sensor.
5) The heat generating composition is taken out from the outer bag.
6) A template (250 mm in length×200 mm in width) having a cavity (80 mm in length×50 mm in width×3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.
With respect to the measurement of the exothermic temperature, the temperature is measured for 10 minutes at a measurement timing of 2 seconds using a data collector, and exothermic rising properties are judged in terms of the temperature after elapsing 3 minutes.
The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.
In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.
With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.
The thickness of the oxygen-containing film of a prescribed amount or the oxygen-containing film of iron powder containing wustite and the amount of wustite are applied to the heat generating composition or the heat generating composition molded body at the time of lamination.
An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50, in particular 0.01 to 20.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

Furthermore, so far as the rising characteristics are not affected, the heat generating composition having a water mobility value falling outside the range of from 0.01 to 20 can contain a water-soluble polymer, a flocculant aid, a flocculant, an agglomeration aid, a dry binder, a dry binding agent, a dry binding material, a sticky raw material, a thickener and an excipient.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together. Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component.

Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The process for producing a heat generating body of the invention is a process for producing a heat generating body having a sectional exothermic part in a stripe form by using a substrate and a covering material, at least one of which is air-permeable; using a heat generating composition which contains, as essential components, an iron powder, a carbon component, a reaction accelerator and water but not contain a flocculant aid, a flocculant, an agglomeration aid, a dry binding material, a dry binding agent, a dry binder, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air and molding the heat generating composition by a die molding method; laminating two or more plural heat generating composition molded bodies in a stripe form at intervals on the planar substrate; folding the covering material in a wavy shape by a fold-providing machine; covering the covering material on the heat generating composition molded body as laminated in a stripe form and provided on the substrate such that crests of the covering material wrap the heat generating composition molded body; traveling a seal-predetermined part while being pressed by a pressing tool; and sealing at least the periphery of the heat generating composition molded body in the MD direction while pressing only both end parts of the covering material.

Furthermore, as the covering material in a wavy shape, one as previously folded in a wavy shape may be used.

The covering material in a wavy shape is not limited so far as its shape is a wavy shape and crests of the waves can wrap and cover the heat generating composition molded body. That is, a covering material capable of keeping the wavy shape without being held by an external force or a covering material which keeps the wavy shape by holding by an external force may be employed. With respect to the covering material which keeps the shape by holding by an external force, the heat generating composition molded body may be wrapped and covered by crests of the waves while keeping the shape by an external force, followed by sealing.

The production process of a covering material in a wavy shape is not limited. Examples thereof include a production process by using a thermoplastic resin and carrying out thermal molding, mechanical embossing, vacuum embossing or other tolerable measure. For example, there is enumerated a method in which a laminate of a nylon-made non-woven fabric and a polyethylene film or a polyethylene film is formed into a wavy shape by thermal molding and used as the covering material in a wavy shape.

Furthermore, in the case of keeping the wavy shape by holding by an external force, there is enumerated a method in which a covering material is passed through a folding machine constituted of a pair of dies having a surface composed of a concavo-convex part, thereby giving the covering material a concavo-convex repeated shape by a combination of a convex surface of one die and a concave surface of the other die, a laminated heat generating composition molded body is wrapped and covered by the resulting covering material as it is, and the periphery of the heat generating composition molded body is further sealed.

The die having a surface composed of a concavo-convex part may be of a fixed type or a mobile type (rotary type). In the case of a fixed type, there is enumerated a folding machine composed of a pair of fixed dies having a surface composed of a concavo-convex part. The mobile type (rotary type) is one for forcing a covering material in and pushing it while rotating. Examples of one die of the pair of dies include a die in which a convex thereof is made of a body of rotation such as bearings; a die composed of a roll in a rotary axis form having a convex in a form of balls on a skewer, and a die for forcing a covering material in a concave and pushing it while moving an endless elastic body such as a rubber ring. Examples of the other die having a concave against the convex include a rotary type die in a drum form having a concavo-convex part. The material quality, the length, the width, the distance, the number, and so on of the convex and the endless elastic body may be determined depending upon the desire. Though there is no limitation regarding the material quality, there are enumerated rubbers, plastics, metals, and the like or expanded bodies or laminates thereof.

In the case of covering the heat generating composition molded body, it is to be hereunder noted that the covering material is a covering material having a wavy shape.

The substrate and the covering material have each a heat seal layer. Furthermore, in the case of carrying out temporary adhesion, it may be achieved by using a heat seal layer or may be further achieved via a sticky layer. In the case of carrying out temporary adhesion via a sticky layer, a sticky layer made of an adhesive is provided on at least one of the heat seal layers, the substrate, the heat generating composition molded body and the covering material are subjected to temporary adhesion in at least the periphery of the heat generating composition molded body via the sticky layer to form a temporarily adhered seal part, and the temporarily adhered seal part is then heat sealed to form a heat seal part. Furthermore, by heat sealing in a width narrower than that of the temporarily adhered seal part and moving the heat generating composition in a region which is not heat sealed within the temporarily adhered seal part, deadhesion is carried out. In this way, the heat generating composition molded body becomes stable; real sealing by heat sealing becomes easy; seal deviation or the like does not occur; a heat seal width with fine lines can be embodied at high speed without causing sealing; and it is possible to section an exothermic part without causing a lowering of exothermic characteristics such as a lowering of exothermic time due to sectioning of the exothermic part.

As a preferred production process of a heat generating body having a sectional exothermic part in which an exothermic part thereof is sectioned according to the molding system of the invention, any molding method using a die may be employed. Examples thereof include a force-through molding method and a cast molding method.

Furthermore, if desired, the heat generating composition or the heat generating composition molded body may be subjected to in-die compression or out-die compression. The "in-die compression" as referred to herein means that the heat generating composition is compressed by flexible rubber rolls or the like while the heat generating composition is present within the die; and the "out-die compression" as referred to herein means that after the heat generating composition leaves from the die to become a heat generating composition molded body, the heat generating composition molded body is compressed by rolls or the like. Though this compression is usually carried out after covering the heat generating composition molded body by an underlay material and/or a covering material, this may not be carried out.

The shape of each of the heat generating composition molded body and the sectional exothermic part is not limited so far as it is in a stripe form. Examples thereof include a rectangular shape, an oblong parallelepiped shape, a cylindrical shape, a semi-pillar shape, a semi-elliptical shape, a semi-cylindrical shape, a pillar shape, and an elliptical shape.

The shape of the heat generating body is not limited. As a planar shape, there are enumerated shapes selected from the group consisting of a circular shape, an elliptical shape, a polygonal shape, a crescent shape, a broad bean-like shape, a cocoon-like shape, a gourd-like shape, a rectangular shape with rounded corners, a square shape with rounded corners, an egg-like shape, a boomerang-like shape, a comma-shaped bead-like shape, and a wing-like shape.

The heat generating body of the invention is produced through the enclosing step and the subsequent sealing step and cutting step and so on. With respect to the sealing step and the cutting step and so on, conventional methods and devices may be properly selected and used.

In the heat generating body, a ratio of the height of a convex of the covering material in a wavy shape to the height of the heat generating composition molded body and a ratio of the width of a convex of the covering material in a wavy shape to the width of the heat generating composition molded body are from 0.7 to 1.0, respectively.

Furthermore, prior to sealing, a ratio of the height of a convex of the covering material in a wavy shape to the height of the heat generating composition molded body is preferably from 0.01 to 1.5; and a ratio of the width of the heat generating composition molded body to the width of a convex of the covering material in a wavy form is preferably from 0.7 to 1.0.

Furthermore, in the sealing step, sealing is not limited so far as sealing can be achieved. Usually, heat sealing or contact bond sealing or a mixture thereof is employed. With respect to the surface of the seal part, any of a plain surface, a pattern in which the cross-section shape thereof is irregular, or a mixture of a plain surface and a pattern in which the cross-section shape thereof is irregular is employable. The "mixture with a pattern" as referred to herein means that the inside of the seal part is plain and the outside is patterned; that the inside of the seal part is patterned and the outside is plain; or that the seal part is partially plain whereas it is partially patterned. Furthermore, the back side may be plain with the front side being patterned, and vice versa. Furthermore, a part or the whole of the pattern may be a multiple pattern. Accordingly, with respect to the seal roll, a plain or patterned roll is used pursuant thereto. Furthermore, a first seal machine of a seal machine may be used as a temporary sealing machine, and after temporary adhesion, heat sealing may be achieved by using a heat seal machine which is a second seal machine. The temporary adhesion may be heat sealing or contact bond sealing via a sticky layer which is constituted of an adhesive. Furthermore, the sealing may be carried out by using a pair of seal rolls or subjected to multiple sealing by using two or more plural pairs of seal rolls. For example, the multiple sealing is double, triple, quadruplet, quintuplet, etc. The seal width may be the same or different and may be properly determined. It is preferable that when the seal speed is high, the number of series is increased. In the case of using heat seal rolls or contact bond seal rolls to which the temperature is applied, the temperature of the pair of rolls may be the same, or the temperature of one roll may be different from that of the other roll.

The "force-through molding method" as referred to herein means a continuous formation method in which by using a molding machine for laminating a heat generating composition molded body having a trimming die shape on a longitudinal substrate by using a trimming die and a rotary seal unit capable of covering the laminate by a longitudinal covering material and sealing (by heat sealing, contact bonding sealing, or heat contact boding sealing) a desired sectioned part and the surroundings of the substrate and the covering material, the surroundings of the heat generating composition molded body and a necessary part of the sectioned part are heat sealed via the seal unit and subjected to a seal treatment.

The "cast molding method" as referred to herein means a molding method in which a heat generating composition molded body is laminated on a longitudinal substrate by filling in a cast having a concave and moving into a substrate. In the case of a continuous molding method, by using a molding machine for laminating a heat generating composition molded body on a longitudinal substrate by filling in a concave and moving into a substrate by a drum-type body of rotation and a rotary seal unit capable of covering the laminate by a longitudinal covering material and sealing (by heat sealing, contact bond sealing, or heat contact bond sealing) a desired sectioned part and the surroundings of the substrate and the covering material, the surroundings of the heat generating composition molded body and a necessary part of the sectioned part are heat sealed via the seal unit and subjected to a seal treatment.

Furthermore, a magnet may be used for molding the heat generating composition of the invention. By using a magnet, it becomes possible to easily achieve accommodation of the heat generating composition in a mold and separation of the molded body from the mold, thereby making it easier to mold a heat generating composition molded body.

Incidentally, the heat generating body may be produced by providing an air-permeable adhesive layer at least between the heat generating composition molded body and the covering material or providing an underlay material such as non-woven fabrics between the heat generating composition molded body and the covering material. In the case of providing an air-permeable adhesive layer at least between the heat generating composition molded body and the covering material, there is no limitation so far as an air-permeable adhesive layer is present at least between the heat generating composition molded body and the covering material. For example, the air-permeable adhesive layer may be provided on the surface of the covering material opposing to the heat generating composition molded body; and the air-permeable adhesive layer may be provided on the heat generating composition molded body or a laminate of the heat generating composition molded body and the substrate and temporarily adhered under pressure or the like between the covering material and the heat generating composition molded body and/or the substrate.

Furthermore, it becomes possible to realize a high-speed production process of a heat generating body by temporarily adhering the substrate and between the heat generating composition molded body laminated on the substrate and the covering material by a sticky layer and then heat sealing the periphery of the heat generating composition molded body and a circumferential seal part which is the surrounding part of the heat generating body.

In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins,such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.

In the case of interposing a heat generating composition molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.

Furthermore, the "deadhesion" as referred to herein means that in the temporary adhering seal part after heat seal, the heat generating composition in a non-heat sealed region is transferred to the foregoing region, thereby releasing the temporary adhesion.

The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

Here, as a process for producing a heat generating body in which an absolute value of a difference between bending resistances in the two directions as substantially intersecting directions becomes maximal, there is enumerated a production process in which a heat generating composition molded body having a size of 120 mm in long side length×6 mm in short side length is prepared by force-through molding; 12 pieces of the heat generating composition molded body are laminated substantially in parallel at equal intervals of 10 mm on a substrate made of a laminate of a nylon-made non-woven fabric and a polyethylene film; an air-permeable covering material made of a laminate of a nylon-made non-woven fabric and a polyethylene-made porous film is covered thereon; the surroundings of 2 mm outside the periphery of each of the heat generating composition molded bodies are heat sealed in a width of 4 mm; the outer surroundings of the heat generating body constituted of the respective heat generating composition molded bodies are further heat sealed in a width of 8 mm; and the outer surroundings of the heat generating body are cut while leaving the heat seal, thereby producing a heat generating body. In a heat generating body as produced by this production process, an absolute value of a difference between bending resistances in the two directions as substantially intersecting directions becomes maximal. Thus, the resulting heat generating body was very excellent in usefulness.

In the heat generating body, a fixing measure-provided heat generating body may be prepared by using a substrate or a covering material in which a fixing measure is provided on at least one exposed surface of a substrate or a covering material, thereby preparing a heat generating body. Alternatively, after heat sealing, an adhesive-provided heat generating body may be produced by providing a hot melt based adhesive in the substrate side by melt blowing or other means in the state prior to cutting, providing a separator thereon and then cutting.

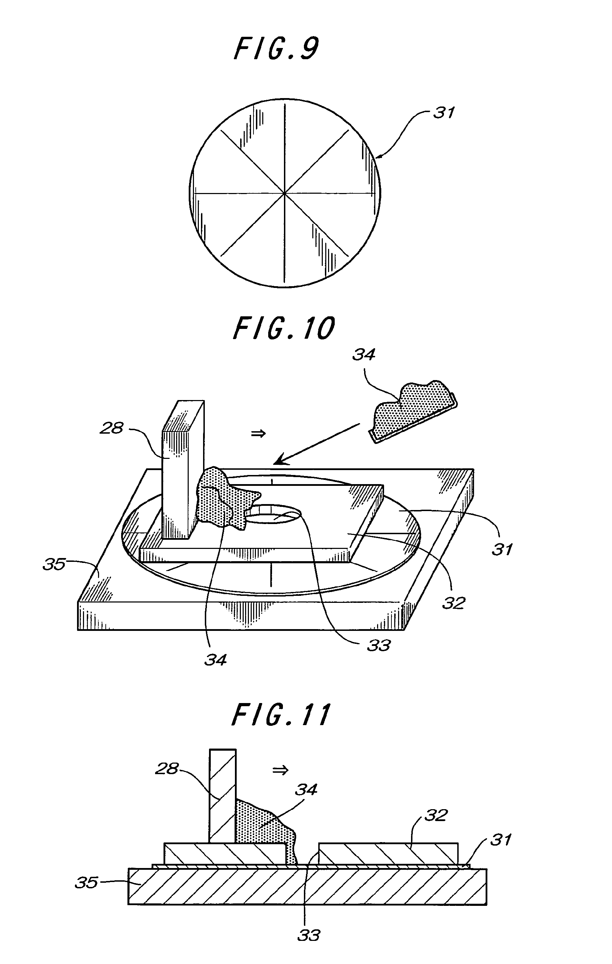

The "water mobility value" as referred to herein is a value showing an amount of surplus water which can transfer to the outside of the heat generating composition in water present in the heat generating composition. This water mobility value will be described below with reference to Figs. 9 to 13.

As shown in Fig. 9, a filter paper 31 of No. 2 (second class of JIS P3801) in which eight lines are drawn radiating from the central point with an interval of 45° is placed on a stainless steel plate 35 as shown in Figs. 10 and 11; a template 32 having a size of 150 mm in length×100 mm in width and having a hollow cylindrical hole 33 having a size of 20 mm in inner diameter×8 mm in height is placed in the center of the filter paper 31; a sample 34 is placed in the vicinity of the hollow cylindrical hole 33; and a stuffer plate 32 is moved on and along the template 32 and inserted into the hollow cylindrical hole 33 while stuffing the sample 34, thereby leveling the sample (force-in die molding).

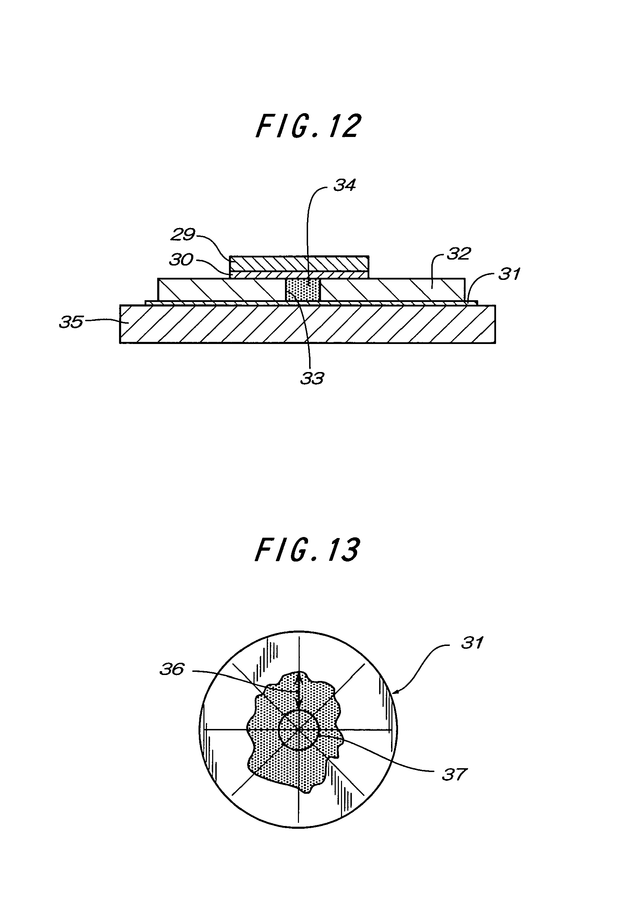

Next, as shown in Fig. 12, a non-water absorptive 70 μm-thick polyethylene film 30 is placed so as to cover the hole 33, and a flat plate 29 made of stainless steel having a size of 5 mm in thickness×150 mm in length×150 mm in width is further placed thereon and held for 5 minutes such that an exothermic reaction is not caused.

Thereafter, a shown in Fig. 13, the filter paper 31 is taken out, and an oozed-out locus of the water or aqueous solution is read as a distance 36 (unit: mm) from a periphery 37 as an edge of the hollow cylindrical hole to an oozed-out tip along the radiating lines. Similarly, a distance 36 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter×8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

$$\text{(Water mobility value)} = \{[\text{Water content value (mm)}]/$$

$$[(\text{Real water content value (mm)})] \times 100$$

With respect to the same sample, five points are measured, and the five water mobility values are averaged, thereby defining an average value thereof as a water mobility value of the sample.

Incidentally, in the foregoing measurement, a filter paper 31 of No. 2 (second class of JIS P3801) in which eight lines are drawn radiating from the central point with an interval of 45° may be provided with a scale mark in each line.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 $\mu$m, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 $\mu$m, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness×200 mm in length×200 mm in width and having a cavity as treated by R5 in four corners of 60 mm in length× 40 mm in width in a central part thereof) and a fixable leveling plate are disposed above a travelable endless belt, and magnets (two magnets having a size of 12.5 mm in thickness× 24 mm in length×24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness×200 mm in length× 200 mm in width is placed on the endless belt of the measurement device, a polyethylene film having a size of 70 $\mu$m in thickness×200 mm in length×200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1. 8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 μm is not present and the number of collapsed pieces of the heat generating composition molded body having a maximum length of from 300 to 800 μm is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (5 mm in thickness×600 mm in length×600 mm in width) of a footed supporting table so as to cover a cavity shape of a molding die.

2) A temperature sensor is placed on the central part the surface of the supporting plate.

3) A polyethylene film (25 μm in thickness×250 mm in length×200 mm in width) as provided with an adhesive layer having a thickness of about 80 μm is stuck onto the supporting plate via a sticky layer such that the center of the polyethylene film is positioned at the sensor.

4) On an underlay plate (280 mm in length×150 mm in width×50 μm to 2 mm in thickness), a polyethylene film (230 mm in length×155 mm in width×25 μm to 100 μm in thickness) is placed such that one end of the polyethylene film is projected by about 20 mm outside the underlay plate and that one end thereof in the length direction is substantially coincident with one end of the underlay plate.

5) A template (230 mm in length × 120 mm in width ×3 mm in thickness) having a cavity (80 mm in length × 50 mm in width × 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

Incidentally, in the invention, it is to be noted that the heat generating composition molded body includes a heat generating composition compressed body.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.

Incidentally, examples of the pain of muscles or bones include acute muscle pain, acute bone pain, acute reference pain, previous muscle pain, previous bone pain, chronic reference pain, and join pain of knee, elbow, etc.

The holding time is not limited but is preferably from 20 seconds to 24 hours, more preferably from one hour to 24 hours, and further preferably from 8 hours to 24 hours.

The holding temperature is preferably from 30 to 50 °C, more preferably from 32 to 50 °C, further preferably from 32 to 43 °C, still further preferably from 32 to 41 °C, and even further preferably from 32 to 39 °C.

The invention will be specifically described below with reference to the Examples, but it should not be construed that the invention is limited thereto.

[Brief Description of the Drawings]

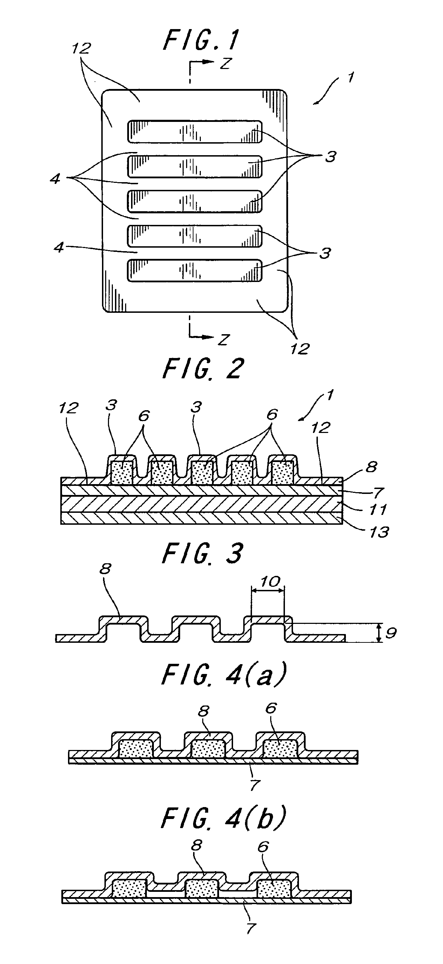

[Fig. 1] is a plan view of an embodiment of the heat generating body of the invention.
[Fig. 2] is a cross-sectional view along the line Z-Z of the same.
[Fig. 3] is a cross-sectional view of a covering material of the same heat generating body.
[Fig. 4] is a cross-sectional view for explaining a covering material and a substrate of the same heat generating body.

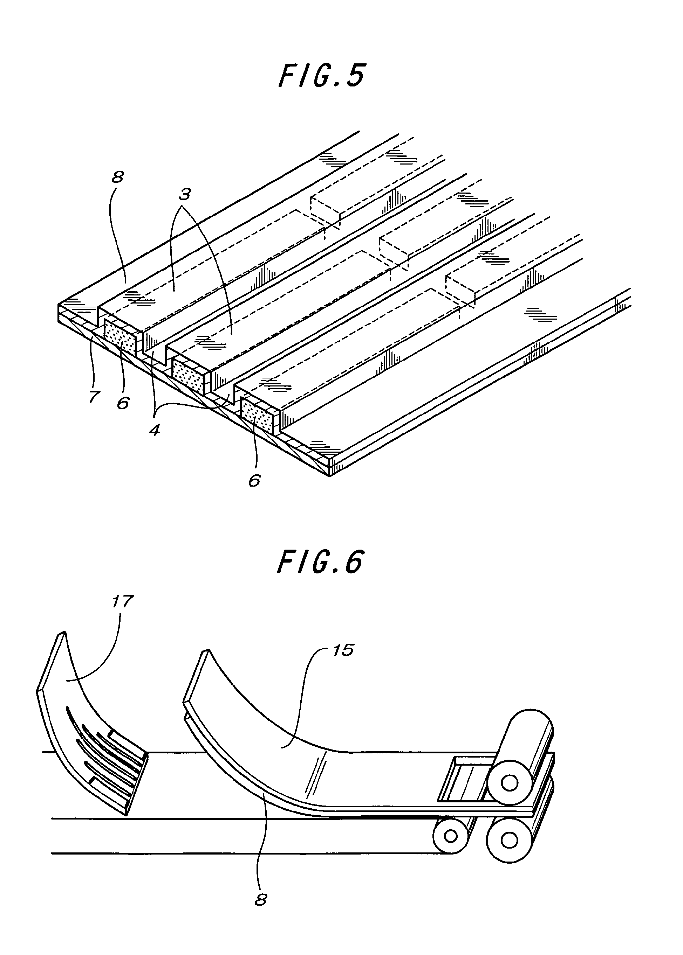

[Fig. 5] is an oblique view for explaining a sectioned part of the same heat generating body.
[Fig. 6] is an oblique view of a device for processing a covering material of the same heat generating body.

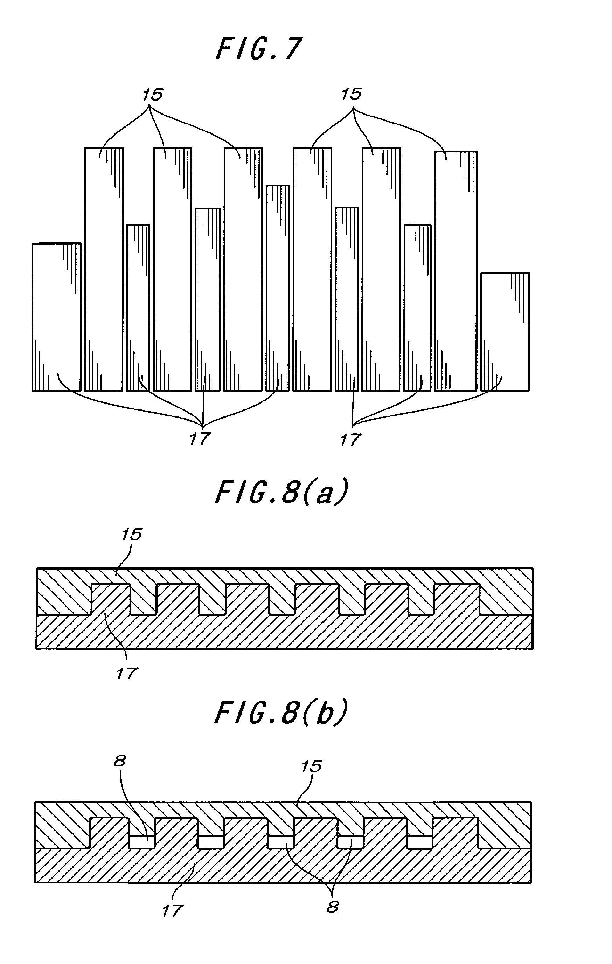

[Fig. 7] is an explanatory drawing of the folded state of a covering material of the same heat generating body.
[Fig. 8] is an explanatory drawing of a device for processing a covering material of the same heat generating body.
[Fig. 9] is a plan view of a filter paper for the measurement of water mobility value in the invention.
[Fig. 10] is an oblique view for explaining the measurement of water mobility value in the invention.
[Fig. 11] is a cross-sectional view for explaining the measurement of water mobility value in the invention.
[Fig. 12] is a cross-sectional view for explaining the measurement of water mobility value in the invention.
[Fig. 13] is a plan view of a filter paper after carrying out the measurement of water mobility value in the invention.

[Description of Reference Numerals and Signs]

1: Heat generating body
3: Heat generating composition (heat generating composition molded body)
4: Seal part (sectioned part)
7: Substrate
8: Covering material
9: Height of convex of covering material in a wavy shape
10: Width of convex of covering material in a wavy shape
12: Outer periphery
15: Concave folding tool
17: Convex folding tool
28: Pushing plate
29: Flat plate
30: Non-water absorptive film (polyethylene film, etc.)
31: Filter paper in which eight lines are drawn radiating from the central point with an interval of 45°
32: Die plate
33: Hole
34: Sample
35: Stainless steel plate
36: Distance to the oozed-out locus of water or aqueous solution
37: Position corresponding to a hollow cylindrical hole on filter paper

[Examples]

(Example 1)

Figs. 1 and 2 each shows the heat generating body of the invention.

A heat generating composition having a water mobility value of 4.3, which is made of a mixture of 100 parts by weight of a reduced iron powder (particle size: not more than 300 $\mu$m), 7.0 parts by weight of active carbon (particle size: not more than 300 $\mu$m), 5.0 parts by weight of a wood meal (particle size: not more than 300 $\mu$m), 0. 8 parts by weight of a water absorptive polymer (particle size: not more than 300 $\mu$m), 0.2 parts by weight of calcium hydroxide, 0.7 parts by weight of sodium sulfite and 11 % salt water, was used as the heat generating composition.

The heat generating composition was molded by force-through molding using a trimming die having five cavities of 5 mm in width×80 mm in length at intervals of 5 mm, thereby providing a heat generating composition molded body 6 constituting five sectional exothermic parts 3 on a polyethylene film 7 provided with a 30 $\mu$m-thick acrylic adhesive layer 11 provided with a separator 13 as illustrated in Fig. 2.

Next, an air-permeable covering material 8 made of a laminate of a nylon-made non-woven fabric having a basis weight of 40 g/m$^2$ on a polyethylene-made porous film was passed through a holding machine composed of a pair of folding tools having a concavo-convex surface and folded in a wavy shape as illustrated in Fig. 3. Then, as illustrated in Fig. 4 (a), valleys of the covering material 8 were pressed onto the substrate 7 by using a folding tool having a concavo-convex surface; the heat generating composition molded body 6 was wrapped and covered within crests of the covering material 8; and as illustrated in Fig. 5, the covering material 8 and the substrate 7 in a corresponding region of the sectioned part 4 were heat sealed.

Fig. 4 (b) shows an embodiment in which a space is present between the substrate 7 and the covering material 8 exclusive of the both end parts.

Furthermore, the covering material may be folded in a wavy shape to wrap and cover the heat generating composition molded body in such a state that the heat generating composition molded body is wrapped and covered within the convex which is a crest of the covering material while leaving a space inclusive of the both end parts. Thereafter, sealing may be achieved as illustrated in Fig. 5.

Next, as illustrated in Fig. 1, the sectioned part 4 which is a seal part of the periphery of the respective heat generating composition molded body 6 was heat sealed in a seal width of 3 mm, thereby preparing the sectional exothermic part 3 as sectioned by the sectioned part 4. Furthermore, a site which is an outer surrounding part 12 of a heat generating body 1 was sealed in a seal width of 8 mm.

Then, the heat generating body 1 having a sectional exothermic part 3 in a stripe form of 98 mm in length×91 mm in width was obtained.

Incidentally, the air permeability of the air-permeable covering material 6 was 400 g/m$^2$/24 hr in terms of a moisture permeability by the Lyssy method. Also, the bending resistance was 20 mm in the long side direction (direction orthogonal to the stripe direction) of the exothermic part and 80 mm or more in the short side direction (stripe direction) , respectively. A ratio of bending resistances in the two directions which are substantially orthogonal to each other was 4 or more. In this way, in the case where the bending resistance in one direction is very high whereas the bending resistance in the direction substantially orthogonal thereto is very low, a feeling for use is very excellent. Furthermore, since this heat generating body 1 can be wound up, it becomes compact and is convenient for accommodation. Incidentally, in the case of the separator-provided heat generating body 1, by using a separator having a low bending resistance, it is possible to wind up it.

The heat generating body 1 was sealed and accommodated in an air-impermeable accommodating body (hereinafter, referred to as "outer bag") and allowed to stand at room temperature for 24 hours. After 24 hours, the heat generating body was taken out from the outer bag and then subjected to an exothermic test. As a result, it was felt warm within 3 minutes, and the warmth was continued for 7 hours. At the same time, curved surface fitness, winding properties and usefulness were evaluated. As a result, the heat generating body was superior in all of these evaluations. Incidentally, a part of the covering material 8 may be placed far from the substrate 7.

(Example 2)

A reaction mixture consisting of 100 parts by weight of a reduced iron powder (particle size: not more than 300 $\mu$m), 5.3 parts by weight of active carbon (particle size: not more than 300 $\mu$m), 5 parts by weight of a wood meal (particle size: not more than 300 $\mu$m), 1.2 parts by weight of a water absorptive polymer (particle size: not more than 300 $\mu$m), 0.2 parts by weight of calcium hydroxide, 0.7 parts by weight of sodium sulfite and 10 parts by weight of 11 % salt water and having a water mobility value of less than 0.01 was used as a heat generating composition. The upper portion of a contact treatment device vessel was opened to air, and the reaction mixture was subjected to self heat generation with stirring under circumstances at 20 °C for 60 seconds, thereby obtaining a heat generating mixture. The reaction mixture had a maximum exothermic temperature of 50 °C. The heat generating mixture had a content of wustite of 10 %. Next, 11 % salt water was added to the heat generating mixture to adjust the water content, thereby obtaining a heat generating composition having a water mobility value of 8.

Next, the heat generating composition was molded by using the substrate 7 and the covering material 8 in the same manner as in Example 1; the heat generating composition molded body 6 in a stripe form as laminated on the substrate 7 was wrapped and covered by the covering material 8 in a wavy shape; a region corresponding to the sectioned part 4 was heat sealed by seal rolls along the MD direction; and the outer peripheries of the substrate 7 and the covering material 8 were heat sealed, thereby preparing a heat generating body composed of seven sectional exothermic parts in a stripe form.

Incidentally, in the preparation of the covering material 8, a folding machine is used for the covering material 8 as illustrate in Fig. 6. In this folding machine, the covering material 8 is interposed between a concave folding tool 15 and a convex folding tool 17, thereby processing the covering material 8 into a concavo-convex form. At this time, the covering material 8 is folded by the folding tools 15 and 17 as illustrated in Fig. 7. Incidentally, Fig. 8(a) shows a cross-sectional view in a combined state of the both folding tools 15 and 17, and the covering material 8 is passed therebetween. Fig. 8(b) shows a cross-sectional view in a combined state thereof with a space being provided exclusive of the both end parts. The resulting heat generating body 1 was sealed and accommodated in an outer bag and allowed to stand at room temperature for 24 hours. After 24 hours, the heat generating body was taken out from the outer bag and then subjected to an exothermic test. As a result, it was felt warm within 3 minutes, and the warmth was continued for 7 hours. At the same time, curved surface fitness, winding properties and usefulness were evaluated. As a result, the heat generating body was superior in all of these evaluations.

(Example 3)

Example 3 is concerned with the production of the heat generating body 1 on a belt conveyor.

On one surface of the folded covering material 8, a hot melt based olefin based adhesive was provided in a cobweb-like state by using a melt blow machine, and a region corresponding to the sectioned part 4 was temporarily adhered and sealed along the MD direction by using temporary adhesion rolls. Next, the same procedures as in Example 2 were followed, except for heat sealing of the outer peripheries of the substrate 7 and the covering material 8 in addition to the foregoing region, thereby preparing the heat generating body 1.

The resulting heat generating body 1 was sealed and accommodated in an outer bag and allowed to stand at room temperature for 24 hours. After 24 hours, the heat generating body was taken out from the outer bag and then subjected to an exothermic test. As a result, it was felt warm within 3 minutes, and the warmth was continued for 7 hours. At the same time, curved surface fitness, winding properties and usefulness were evaluated. As a result, the heat generating body was superior in all of these evaluations.

**Claims**

1. A heat generating body having an exothermic part in a stripe form of an integral structure made of a sectional exothermic part as formed by providing plural heat generating composition molded bodies in a stripe form at intervals on a planar substrate, covering a covering material as folded in a wavy shape thereon such that crests of the covering material wrap the heat generating composition molded bodies and sealing the peripheries of the heat generating composition molded bodies and a sectioned part as the seal part, **characterized in that**:

   1) the heat generating composition molded body is constituted of a moldable heat generating composition,
   2) the moldable heat generating composition contains, as essential components, an iron powder, a carbon component, a reaction accelerator and water but not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air,
   3) a ratio of the height of a convex of the covering material in a wavy shape to the height of the heat generating composition molded body and a ratio of the width of a convex of the covering material in a wavy shape to the width of the heat generating composition molded body are from 0.7 to 1.0, respectively,
   4) a part of the sectional exothermic part is air-permeable, and
   5) the surroundings of the heat generating body are sealed.

2. The heat generating body as set forth in claim 1, **characterized in that** a volume of the heat generating composition molded body is from 0.1 to 30 cm$^3$; a ratio of the capacity of the sectional exothermic part to the volume of the heat generating composition molded body is from 0.6 to 1.0; a maximum height of the sectional exothermic part is from 0.1 to 10 mm; a width of the sectioned part is from 0.3 to 50 mm; and the seal part is formed by heat sealing.

3. The heat generating body as set forth in claim 1, **characterized in that** the moldable heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

4. The heat generating body as set forth in claim 1, **characterized in that** in the heat generating body, the heat generating composition molded body is at least compressed.

5. The heat generating body as set forth in claim 1, **characterized in that** a fixing measure is provided on at least a part of the exposed surface of the heat generating body.

6. A process for producing a heat generating body,
   **characterized in that**:

   1) at least one of a substrate and a covering material is air-permeable,
   2) a composition which contains, as essential components, an iron powder, a carbon component, a reaction accelerator and water but does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20 and has moldability due to the surplus water as a connecting substance, with the water in the heat generating composition not functioning as a barrier layer, and is capable of causing a heat reaction upon contact with air is used as a moldable heat generating composition,
   3) plural heat generating composition molded bodies as formed by molding the moldable heat generating composition are provided in a stripe form at intervals on the substrate,
   4) the covering material is formed in a wavy shape by a fold-providing machine and covered such that crests of the covering material come into contact with the heat generating molded body,
   5) the substrate covered with covering material is conveyed while a part to be sealded is pressed by a pressing tool, and
   6) at least the periphery of the heat generating composition molded body is sealed in the MD direction while pressing both end parts of the covering material.

7. The process for producing a heat generating body as set forth in claim 6, **characterized in that** a volume of the heat generating composition molded body is from 0.1 to 30 cm$^3$; a ratio of the capacity of the sectional exothermic part to the volume of the heat generating composition molded body is from 0.6 to 1.0; a maximum height of the sectional exothermic part is from 0.1 to 10 mm; and a width of the sectioned part is from 0.3 to 50 mm.

8. The process for producing a heat generating body as set forth in claim 7, **characterized in that** the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

## FIG. 1

## FIG. 2

## FIG. 3

## FIG. 4(a)

## FIG. 4(b)

## FIG.5

## FIG.6

# FIG. 7

*15*              *15*

*17*              *17*

# FIG. 8(a)

*15*

*17*

# FIG. 8(b)

*8*              *15*

*17*              *8*

# FIG.9

# FIG.10

# FIG.11

# FIG.12

# FIG.13

## INTERNATIONAL SEARCH REPORT

International application No.

PCT/JP2005/013004

| A. CLASSIFICATION OF SUBJECT MATTER |
|---|
| Int.Cl⁷ A61F7/08, C09K5/16 |

According to International Patent Classification (IPC) or to both national classification and IPC

| B. FIELDS SEARCHED |
|---|
| Minimum documentation searched (classification system followed by classification symbols) |
| Int.Cl⁷ A61F7/08, C09K5/16 |

Documentation searched other than minimum documentation to the extent that such documents are included in the fields searched

| Jitsuyo Shinan Koho | 1922-1996 | Jitsuyo Shinan Toroku Koho | 1996-2005 |
|---|---|---|---|
| Kokai Jitsuyo Shinan Koho | 1971-2005 | Toroku Jitsuyo Shinan Koho | 1994-2005 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| Y<br>A | JP 2003-334212 A (Maikoru Kabushiki Kaisha),<br>25 November, 2003 (25.11.03),<br>Full text; Fig. 2<br>(Family: none) | 1,3-5<br>6,8 |
| Y<br>A | JP 2003-336042 A (Maikoru Kabushiki Kaisha),<br>28 November, 2003 (28.11.03),<br>Full text; Figs. 1 to 16<br>& US 2004/0217325 A    & EP 1516900 A<br>& WO 2003/097764 A1    & CA 2439044 A<br>& CN 1496395 A | 1,3-5<br>6,8 |
| A | JP 2002-155273 A (Kaoru USUI),<br>28 May, 2002 (28.05.02),<br>Full text; Figs. 1 to 14<br>(Family: none) | 1,3-5,6,8 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 September, 2005 (14.09.05) | 11 October, 2005 (11.10.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

<table>
<tr><td colspan="3" align="center">INTERNATIONAL SEARCH REPORT</td><td>International application No.<br>PCT/JP2005/013004</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | JP 2004-24671 A  (Maikoru Kabushiki Kaisha),<br>29 January, 2004 (29.01.04),<br>Page 4, lines 40 to 44; page 5, lines 31 to<br>41; page 8, lines 11 to 34; Fig. 15<br>(Family: none) | 1-5<br>6-8 |
| Y | CD-ROM of the specification and drawings<br>annexed to the request of Japanese Utility<br>Model Application No. 5759/1992(Laid-open<br>No. 58123/1993)<br>(Shozo NAKAJIMA),<br>03 August, 1993 (03.08.93),<br>Full text; Fig. 1<br>(Family: none) | 1 |
| Y | JP 3007467 U  (Kiribai Kagaku Kabushiki<br>Kaisha),<br>14 February, 1995 (14.02.95),<br>Page 9, lie 17 to page 10, line 14;<br>Figs. 1 to 8<br>(Family: none) | 1 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4293989 A
- JP 6343658 A
- JP 59189183 A
- WO 0013626 A
- JP 9075388 A
- JP 60101448 A
- JP 9276317 A
- JP 11299817 A
- JP 6026829 A
- JP 2000288008 A
- JP 11508314 T
- JP 11508786 T

- JP 2002514104 T
- JP 2002200108 A
- JP 10265373 A
- JP 9087173 A
- JP 6145050 A
- JP 6199660 A
- JP 10279466 A
- JP 10182408 A
- JP 3161605 B
- JP 2001507593 T
- JP 7194641 A